# EUROPÄISCHE PATENTANMELDUNG

(11) **EP 3 524 976 A1**
(43) Veröffentlichungstag der Anmeldung: **14.08.2019**
(21) Anmeldenummer: 18155871.9
(22) Anmeldetag: 08.02.2018
(51) Int. Cl.: G01N 33/15, G01N 3/40

(54) **VORRICHTUNG UND VERFAHREN ZUR KALIBRIERUNG VON GESCHWINDIGKEIT UND BRUCHKRAFT EINES TABLETTENPRÜFGERÄTS**

(71) Anmelder: Erweka GmbH, 63225 Langen (DE)
(72) Erfinder: MÜLLER, Werner G., 63225 Langen (DE); BOZKURT, Levent, 61191 Rosbach (DE)
(74) Vertreter: Dr. Meyer-Dulheuer & Partners LLP

(57) **Zusammenfassung**

Vorrichtung (1) und Verfahren zum Kalibrieren eines Tablettenprüfgeräts (8), mit einem Messgerät (2) zur Erfassung von Geschwindigkeit und/oder Bruchkraft einer Bruchbacke (11) des Tablettenprüfgeräts (8), einer Rechnereinheit (5) zur Auswertung der bei der Erfassung erhaltenen Daten, einer Übertragungsverbindung (7) zum Tablettenprüfgerät (8) und Verbindungsmitteln (4) zwischen dem Messgerät (2), der Rechnereinheit (5) und der Übertragungsverbindung (7).

## Beschreibung

Die Erfindung betrifft eine Vorrichtung zum Ausrüsten eines Tablettenprüfgeräts mit einer Kalibriereinheit, durch die sich die Geschwindigkeit oder die Bruchkraft einer Bruchbacke von Tablettenprüfgeräten zur Bestimmung der Bruchhärte von Tabletten kalibrieren und justieren lässt. Durch diese Vorrichtung lassen sich die Geschwindigkeit einer Bruchbacke und die anzulegende Bruchhärte in regelmäßigen Abständen kalibrieren, so dass die Erfordernisse der modernen Qualitätskontrolle an pharmazeutische Messinstrumente erfüllt werden können. Die Vorrichtung wird zum zeitweisen Ausrüsten eines Tablettenprüfgeräts für die Kalibrierung genutzt, indem die Vorrichtung nur zur Kalibrierung mit dem Tablettenmessgerät verbunden wird, so dass die sperrige und raumfüllende Vorrichtung während des Normalbetriebes von dem Tablettenprüfgerät getrennt gelagert werden kann. Die Erfindung betrifft ausserdem ein Verfahren zum Kalibrieren eines Tablettenprüfgeräts mit der genannten Vorrichtung.

Tabletten müssen vor dem Einbringen in den Handel auf ihre Bruchfestigkeit geprüft werden. Hierzu kommen üblicherweise Tablettenprüfgeräte zum Einsatz, die in rascher Folge Tabletten auf ihre Bruchhärte überprüfen. Diese bestehen aus einer Prüfkammer, einer Gegenbacke und einer beweglichen Bruchbacke, mit der die Tablette zwischen der Gegenbacke und der Bruchbacke eingeklemmt werden kann. Außerdem enthalten die Tablettenprüfgeräte noch eine Antriebsvorrichtung zum Antrieb der Bruchbacke und Messeinrichtungen zum Messen der Geschwindigkeit der Bruchbacke und der anzulegenden Bruchkraft. Oft werden die Tablettenprüfgeräte auch zum Ausmessen der äußeren Abmessungen von Tabletten genutzt, indem die Bruchbacke eines Tablettenprüfgeräts nicht bis zum Zerbrechen der Tablette auf diese zubewegt wird, sondern durch Voreinstellung einer aufzuwendenden Kraft angehalten wird und die zurückgelegte Wegstrecke der Bruchbacke bestimmt und zur Berechnung der Länge, Breite oder des Durchmessers einer Tablette genutzt wird.

Beispiele für Tablettenprüfgeräte sind aus dem Stand der Technik bekannt. Das Dokument DE102015114600B3 beschreibt eine Vorrichtung und ein Verfahren zur Prüfung von Tabletten, wobei die Vorrichtung eine Prüfkammer, eine Bruchbacke und eine ihr gegenüberliegende Gegenbacke und ein vorzugsweise in einem rechten Winkel zu der Gegenbacke angeordnetes Widerlager aufweist, und wobei die Vorrichtung über einen beweglichen Blechstreifen verfügt, der dazu eingerichtet ist, eine Tablette zur Prüfung zu positionieren und auszurichten, und die Längsachse des beweglichen Blechstreifens in einem Winkel von unter 90° zur Längsachse der Bewegungsrichtung der Bruchbacke ausgerichtet ist und die Bewegungsrichtung des Blechstreifens der Längsachse des Blechstreifens entspricht, und durch die Bewegung des Blechstreifens in eine Richtung die Tablette entlang des Widerlagers und durch Bewegung des Blechstreifens in die entgegengesetzte Richtung entlang der Gegenbacke positionierbar ist.

Die Prüfkammer besteht bei Tablettenprüfgeräten häufig aus einer langgestreckten Positionierungsfläche, in die die Tabletten nach einer Vereinzelung zur einzelnen Messung hineingegeben werden. Hierzu werden diese zunächst durch ein Vereinzelungsgerät vereinzelt und dann einzeln auf einer von einer Gegenbacke und einer Bruchbacke umfassten Messfläche positioniert. Dann wird die Bruchbacke auf die Gegenbacke zubewegt, wobei sich die vereinzelte zu messende Tablette zwischen der Bruchbacke und der Gegenbacke befindet und schließlich zwischen diesen eingeklemmt wird. Das Zubewegen der Bruchbacke geschieht dabei mit einer langsamen Geschwindigkeit, damit die Tablette in einer gewünschten Position zum Messen eingeklemmt wird. Nach dem Einklemmen lässt man die Bruchkraft dann mit einem vorbestimmten Gradienten ansteigen, damit die Bruchkraft langsam bis zum Zerbrechen der Tablette erhöht wird. Oft werden mit der Bruchfestigkeit auch die äußeren Abmessungen der Tablette durch Anhalten der Bruchbacke mit einer vorbestimmten Bruchkraft vor dem Zerbrechen der Tablette mitbestimmt.

Die Messung der Abmessungen und der Bruchfestigkeit der Tabletten wird dabei nach genau vorbestimmten und genormten Verfahrensschritten vorgenommen. Diese Messungen sind genormt und sind in der Fachliteratur hinterlegt. Ein Beispiel hierzu ist das Deutsche Arzneibuch. Ein weiteres Beispiel, welches die Messung der Abmessungen und der Bruchfestigkeit von Tabletten beschreibt, ist das USamerikanische Arzneimittelbuch "The United States Pharmacopeial Convention", Ausgabe USP 40 vom 01. Dezember 2017, Kapitel 1217 "General Information", Seiten 1750 bis 1752, Internet: http://app.uspnf.com/uspnf/pub/index?usp=40&nf=35&s=2&officialOn=December%2 01,%202017. Dort wird beschrieben, dass eine Kalibrierung der Messgeräte unerlässlich ist, da die Messung der Tablette reproduzierbar sein muss. Der Anstieg der Bruchkraft muss konstant sein, da andernfalls ein zu plötzlicher Anstieg von Druckkräften erfolgt, der zum unkontrollierten Zerquetschen der Tablette und zum Bruch der Tablette durch Schereinwirkung führt. Kalibriert werden müssen insbesondere die Geschwindigkeit, mit der die Bruchbacke auf die Tablette zubewegt wird, und die Bruchkraft, die zum reproduzierbaren Zerbrechen der Tablette erforderlich ist.

Tablettenprüfgeräte, die sich im Handel befinden und die beim Kunden aufgestellt sind, müssen deshalb in regelmäßigen Abständen kalibriert werden, um weiterhin zuverlässige Ergebnisse zu liefern. Typische Intervalle, in denen eine Kalibrierung vorgenommen wird, sind beispielsweise Jahresfristen.

Die Kalibrierung wird bisher und im Stand der Technik mit Werkzeug durchgeführt, welches stets zusammengestellt werden muss, wenn die erneute Kalibrierung eines Tablettenprüfgeräts ansteht. Dies ist in der Regel aufwendig und mit einer langen Vorbereitungsdauer verbunden. Deshalb wäre es von großem Vorteil, wenn ein fertiges Gerät bereitstünde, mit dem ein Tablettenprüfgerät durch einfaches Verbinden mit einem Kalibriergerät oder durch einfaches Aufsetzen des Kalibriergerätes für einen Kalibrierungsvorgang vorbereitet werden kann. Eine stationäre Einrichtung, mit der ein Tablettenprüfgerät zum Kalibrieren augestattet wird, ist in der Regel nicht erwünscht, da handelsübliche Tablettenprüfgeräte handlich und transportabel sind. Eine stationäre Kalibriereinrichtung, die mit dem Tablettenprüfgerät verbunden wäre, würde den Raumbedarf eines vorhandenen Tablettenprüfgerätes in unerwünschter Weise erhöhen.

Es besteht deshalb die Aufgabe, eine Vorrichtung bereitzustellen, durch die ein Tablettenprüfgerät in einfacher Weise für eine einzige Kalibrierung innerhalb eines Kalibrierungsintervalls ausgerüstet werden kann. Die vorliegende Erfindung löst diese Aufgabe durch ein Kalibriergerät, welches mit mindestens einem Messgerät zur Kalibrierung, einer Rechnereinheit, einem Übertragungsmittel zu dem Tablettenprüfgerät, und Verbindungsmitteln zwischen dem Messgerät, der Rechnereinheit und dem Übertragungsmittel ausgestattet ist.

Durch das Verbinden eines solchen Kalibriergerätes mit dem Tablettenprüfgerät kann das Tablettenprüfgerät einfach und ohne großen Zeitverlust zur Kalibrierung ausgerüstet werden. Durch die vorliegende Erfindung lässt sich ein Tablettenprüfgerät einfach und zeitsparend mit einer Vorrichtung zur Kalibrierung versehen, die nur während der Kalibrierung innerhalb des vorgesehenen Kalibrierungsintervalls zum Einsatz gelangt. Während des Normalbetriebes wird die sperrige und raumfüllende Vorrichtung zur Kalibrierung von dem Tablettenprüfgerät getrennt gelagert. Ausserdem werden durch das Tablettenprüfgerät Fehlerquellen vermieden, weil die Kalibrierung mit dem vorbestimmten Aufbau der Kalibriervorrichtung durchgeführt wird.

Die Rechnereinheit besteht dabei aus einem Rechner, der die zur Kalibrierung erforderlichen Daten enthält, gegen die die Kalibrierung dann erfolgt. Die Daten wurden vorher, beispielweise durch Programmierung des Rechners, in diesen eingespeist. Die Steuerung der Messgeräte und der Rechnereinheit während der Kalibrierung erfolgt über die Anzeigeeinheit und die Eingabeeinheit des Tablettenprüfgeräts. Durch die Vorrichtung der vorliegenden Erfindung können die Erfordernisse der modernen Qualitätskontrolle an pharmazeutische Messinstrumente auf einfache Weise erfüllt werden.

Üblicherweise werden mit diesem Kalibriergerät die Geschwindigkeit, mit der die Bruchbacke auf die Tablette zubewegt wird, und die erforderliche Bruchkraft, die zum Zerbrechen einer Tablette erforderlich ist, kalibriert. Es ist jedoch im Rahmen der Erfindung auch möglich, nur die Geschwindigkeit der Bruchbacke oder nur die Bruchkraft, die zum Zerbrechen einer Tablette erforderlich ist, zu kalibrieren.

Beansprucht wird insbesondere eine Vorrichtung zur Kalibrierung eines Tablettenprüfgeräts, welche ein Messsystem von Geschwindigkeit oder Bruchkraft einer Bruchbacke in einem Tablettenprüfgerät bildet, umfassend
- mindestens ein Messgerät zur Erfassung von Geschwindigkeit oder Bruchkraft einer Bruchbacke des Tablettenprüfgeräts,
- eine Rechnereinheit zur Erfassung und Auswertung der bei der Erfassung erhaltenen Daten,
und welche dadurch gekennzeichnet ist, dass
- diese eine Übertragungsverbindung zu einem Tablettenprüfgerät, welches eine Anzeigeeinheit und eine Eingabeeinheit enthält, umfasst, und
- Verbindungsmittel zwischen dem oder den Messgeräten, der Rechnereinheit und der Übertragungsverbindung, so dass
- die Vorrichtung über die Übertragungsverbindung mit dem Tablettenprüfgerät verbindbar ist, und damit die über die Messgeräte erhaltenen Daten über die Übertragungsverbindung an das Tablettenprüfgerät übertragbar sind und dadurch auf dessen Anzeigeeinheit sichtbar und über dessen Eingabeeinheit bearbeitbar sind, und
- die Rechnereinheit zur Auswertung der bei der Messung von Geschwindigkeit oder Bruchkraft erhaltenen Daten nutzbar ist und dadurch der Antrieb der Bruchbacke des Tablettenprüfgeräts steuerbar und kalibrierbar ist.

In einer Ausführungsform der Erfindung handelt es sich bei dem oder den Messgeräten um eine Wegstreckenerfassungseinheit, eine Kraftmessdose oder um beides. Die Wegstreckenerfassungseinheit kann beispielsweise ein optisches Messgerät sein. Ein optisches Messgerät wiederum kann beispielsweise ein Lasermessgerät sein. Weitere optische Messgeräte und Kraftmessdosen zur Bestimmung der bei einer Bruchhärte aufgewendeten Kraft werden beispielhaft beschrieben in der Patentanmeldung WO2011035818A1. Eine Wegstreckenerfassungseinheit kann in einer einfachen Ausführungsform eine Reihe von elektrischen Kontakten sein, die zur Messung der Wegstrecke eines sich vorbeibewegenden Gegenstandes dienen. Die elektrischen Kontakte sind dann aufeinanderfolgend angeordnet und entsprechen in der Zahl der zurückgelegten Wegstrecke. Zum Messen der Wegstrecke werden die Kontakte aufeinanderfolgend von der Bruchbacke beim Zurücklegen des Weges durch einen Gegenkontakt betätigt, wodurch die Wegstrecke bestimmt wird.

In einer weiteren Ausführungsform der Erfindung handelt es sich bei der Übertragungsverbindung zu einem Tablettenprüfgerät um eine USB-Steckverbindung. Dadurch wird insbesondere die Vorrichtung mit dem Tablettenprüfgerät verbunden. Durch einen USB-Stecker, wie dieser in der Computertechnik weitverbreitet und üblich ist, ist eine schnelle und genormte Verbindungsmöglichkeit zwischen der Vorrichtung und dem Tablettenprüfgerät möglich. Einsetzbar sind auch andere Übertragungsvorrichtungen wie beispielsweise drahtlose Übertragungsvorrichtungen.

Der Aufnahmepol für die USB-Steckverbindung sitzt an dem Tablettenprüfgerät an beliebiger Stelle, ist jedoch bevorzugt mit der Anzeigeeinheit des Tablettenprüfgerätes verbunden. Es versteht sich, dass die Rechnereinheit der Kalibriervorrichtung der vorliegenden Erfindung mit dem Rechner des Tablettenprüfgerätes kompatibel ist.

In einer weiteren Ausführungsform der Erfindung handelt es sich bei den Verbindungsmitteln zwischen dem oder den Messgeräten, der Rechnereinheit und der Übertragungsverbindung um elektrische Kabel.

Die Rechnereinheit ist bevorzugt eine Rechnereinheit, die die bei einer Messung erhaltenen Daten zu Geschwindigkeit oder Bruchkraft selbstständig auswertet und an die Anzeigeeinheit und die Eingabeeinheit des Tablettenprüfgeräts überträgt. Rechnereinheiten zur Kalibrierung von Tablettenmessgeräten sind im Stand der Technik bekannt, ebenso Rechenprogramme, mit denen diese Rechnereinheiten betrieben werden.

Umfasst die erfindungsgemäße Vorrichtung mehrere Messgeräte und enthält das Tablettenprüfgerät bereits ein Rechenprogramm zur Kalibrierung, so kann es sich bei der Rechnereinheit in einer einfachen Ausführungsform auch um eine Verteilerdose handeln, die die bei einer Messung erhaltenen Daten zu Geschwindigkeit oder Bruchkraft an den Rechner des Tablettenprüfgeräts überträgt.

In einer Ausführungsform der Erfindung ist mit der Vorrichtung eine Kalibrierung von Geschwindigkeit oder Bruchkraft einer Bruchbacke des Tablettenprüfgeräts möglich. Im ersten Fall enthält die Vorrichtung eine Wegstreckenerfassungseinheit und im zweiten Fall eine Kraftmessdose. In einer bevorzugten Ausführungsform der Erfindung ist mit der Vorrichtung der vorliegenden Erfindung jedoch eine Kalibrierung von Geschwindigkeit und Bruchkraft möglich. In diesem Fall enthält die Vorrichtung eine Wegstreckenerfassungseinheit und eine Kraftmessdose. Die Rechnereinheit ist dabei so ausgestattet, dass entweder die Kalibrierung der Geschwindigkeit oder die Kalibrierung der Bruchkraft oder die Kalibrierung der Geschwindigkeit und der Bruchkraft durchführbar sind. Die Rechnereinheit enthält dann die entsprechenden Programme.

Mit der erfindungsgemäßen Vorrichtung ist statt oder zusätzlich zu einer Kalibrierung auch eine Speicherung oder Aufzeichnung der erhaltenen Daten von Geschwindigkeit oder Bruchkraft oder beidem möglich. In diesem Fall enthält die Rechnereinheit entsprechende Programme zur Speicherung oder Aufzeichnung.

In einer Ausführungsform der Erfindung ist die Vorrichtung zur Kalibrierung auf ein Tablettenprüfgerät aufsetzbar oder aufschraubbar. Dadurch lässt sich diese leicht und in kurzer Zeit auf das Tablettenmessgerät aufmontieren. Dadurch können insbesondere die Messgeräte zu Geschwindigkeit und Bruchkraft leicht auf das Tablettenmessgerät aufmontiert werden. Das Aufschrauben dieser Messgeräte auf das Tablettenmessgerät kann beispielhaft durch Schraubverbindungen ernöglicht werden. Das Aufsetzen dieser Messgeräte auf das Tablettenmessgerät kann beispielhaft durch Steckverbindungen ermöglicht werden.

Beansprucht wird auch ein Verfahren zur Kalibrierung von Tablettenmessgeräten, durch welches ein Tablettenmessgerät mit der hier beschriebenen Vorrichtung kalibriert wird. Durch dieses Verfahren wird die oben beschriebene Vorrichtung zur Kalibrierung mit einem Tablettenmessgerät verbunden, die Geschwindigkeit oder die Bruchkraft der Bruchbacke wird kalibriert, und die Vorrichtung zur Kalibrierung wird wieder entfernt.

Beansprucht wird insbesondere ein Verfahren zur Kalibrierung von Geschwindigkeit oder Bruchkraft der Bruchbacke eines Tablettenprüfgeräts, in dem
- in einem Tablettenprüfgerät eine zur Messung vorgesehene Tablette in eine Prüfkammer zwischen einer Gegenbacke und einer Bruchbacke des Tablettenprüfgeräts gegeben wird,
- die Bruchbacke mit einer bestimmten Geschwindigkeit auf die Gegenbacke des Tablettenprüfgeräts zufährt,
und welches dadurch gekennzeichnet ist, dass
- das Tablettenprüfgerät mit einer Vorrichtung zur Kalibrierung wie oben beschrieben, welche ein mindestens ein Messgerät zur Erfassung von Geschwindigkeit oder Bruchkraft einer Bruchbacke, eine Rechnereinheit, eine Übertragungsverbindung, und Verbindungsmittel zwischen dem oder den Messgeräten, der Rechnereinheit und der Übertragungsverbindung umfasst, verbunden wird,
- bei der Messung die von der Bruchbacke zurückgelegte Wegstrecke pro Zeiteinheit oder die Bruchkraft erfasst wird und dadurch die Geschwindigkeit oder die Bruchkraft der Bruchbacke gemessen und gegen gespeicherte Werte kalibriert wird, und
- das Tablettenprüfgerät nach der Kalibrierung wieder von der Vorrichtung zur Kalibrierung getrennt wird.

Unter Bruchkraft wird dabei die zum Zerbrechen der Tablette aufzuwendende Bruchkraft verstanden. In einer bevorzugten Ausführungsform der Erfindung werden durch das Verfahren sowohl die Bruchkraft als auch die Geschwindigkeit der Bruchbacke gemessen und gegen gespeicherte Werte kalibriert.

In einer weiteren Ausführungsform der Erfindung wird die Kalibrierung gegen Kalibrierungswerte als Referenzwerte durchgeführt, die in der Rechnereinheit gespeichert sind. Hierzu ist die Rechnereinheit vorher mit geeigneten Werten zur Kalibrierung programmiert worden. Es ist im Rahmen der Erfindung auch denkbar, dass die Kalibrierung anhand von Daten durchgeführt wird, die in der Rechnereinheit des Tablettenprüfgerätes enthalten sind. In diesem Fall werden mit der oben beschriebenen Vorrichtung nur die Messgeräte, die dann über die Verteilermessdose und die Verbindungsmittel über die Übertragungsverbindung mit dem Tablettenprüfgerät verbunden werden, auf das Tablettenprüfgerät aufgesetzt. Weiterhin ist es im Rahmen der Erfindung möglich, dass die Kalibrierungswerte, gegen die kalibriert wird, in einem externen Rechner gespeichert sind. Der externe Rechner kann beispielsweise ein PC sein. Die Vorrichtung der vorliegenden Erfindung kann dann mit dem PC und dem Tablettenprüfgerät in beliebiger Art und Weise verbunden werden, um eine Kalibrierung durchzuführen. So kann beispielsweise die Vorrichtung zur Kalibrierung über USB-Stecker als Übertragungsverbindungen mit einem PC, in dem die Kalibrierungswerte gespeichert sind, und mit dem Rechner des Tablettenprüfgeräts, mit dem üblicherweise Messungen vorgenommen werden, zur Kalibrierung verbunden werden, während die Messgeräte der Vorrichtung zur Kalibrierung auf das Tablettenprüfgerät aufgesetzt werden.

In einer Ausführungsform des Verfahrens wird die Geschwindigkeit der Bruchbacke durch die Kalibrierung auf einen Wert von 0,1 mm/Sekunde bis 3,0 mm/Sekunde eingestellt. Dieser Wert hat sich bei der Messung von Tabletten als sehr vorteilhaft erwiesen.

Bei der Kalibrierung des Tablettenprüfgeräts nach dem erfindungsgemäßen Verfahren erfolgt in einer Ausführungsform des Verfahrens bei der Kalibrierung eine Messung auf Linearitätsabweichungen. Diese wird bei der Kalibrierung von Tablettenprüfgeräten oftmals durchgeführt, und ist beispielsweise beschrieben in dem Dokument "The United States Pharmacopeial Convention", Ausgabe USP 40 vom 01. Dezember 2017, Kapitel 1217 "General Information", Seiten 1750 bis 1752, Internet: http://app.uspnf.com/uspnf/pub/index?usp=40&nf=35&s=2&officialOn=December%2 01,%202017. Die Messung auf Linearitätsabweichungen erfolgt, indem innerhalb der Wegstrecke der Bruchbacke eine Messung von bis zu drei Punkten in einer Geraden durchgeführt wird.

Die Vorrichtung zur Kalibrierung von Tablettenprüfgeräten kann während der Kalibrierung auf das Tablettenprüfgerät aufgesetzt oder aufgeschraubt werden. Hierzu enthält die Vorrichtung zur Kalibrierung von Tablettenprüfgeräten beispielsweise Schraub- oder Steckverbindungen.

Weiterhin wird mit der oben beschriebenen Vorrichtung üblicherweise und in einer bevorzugten Ausführungsform eine Kalibrierung des Tablettenprüfgerätes durchgeführt. Es ist im Rahmen der Erfindung aber auch möglich, lediglich eine Aufzeichnung oder Speicherung der erhaltenen Daten durchzuführen. Diese können beispielsweise später ausgewertet werden oder grafisch, beispielsweise durch einen Bildschirm, dargestellt werden. Diese können auch für spätere Zwecke, beispielsweise für eine erneute Kalibrierung, verwendet werden.

Beansprucht wird auch ein Verfahren zur Messung von Tabletten, bei dem eine oben beschriebene Vorrichtung zum Einsatz kommt. Beansprucht wird also insbesondere ein Verfahren zur Messung von Tabletten, welches dadurch gekennzeichnet ist, dass dabei ein mit einer oben beschriebenen Vorrichtung der vorliegenden Erfindung kalibriertes Tablettenmessgerät zum Einsatz kommt. Durch ein so kalibriertes Tablettenmessgerät wird dann insbesondere die Bruchhärte von Tabletten bestimmt.

Im Rahmen des Verfahrens der vorliegenden Erfindung können nach der Kalibrierung der Geschwindigkeit und der Bruchkraft der Bruchbacke auch die Abmessungen der Tablette bestimmt werden. Dies ist vorteilhaft möglich, weil zur Bestimmung der Abmessungen der Tablette die Bruchbacke mit einer vorbestimmten Geschwindigkeit und einer vorbestimmten Bruchkraft auf die Tablette zubewegt werden muss. Bei den Abmessungen einer Tablette kann es sich beispielhaft um die Länge, die Breite oder den Radius der Tablette handeln.

Zur Bestimmung der Abmessungen wird die Bruchbacke mit einer vorbestimmten und genau kalibrierten Geschwindigkeit auf die Tablette zubewegt. Kurz vor dem Erreichen der Tablette wird die Geschwindigkeit der Bruchbacke verlangsamt. Die Bruchbacke fährt dann mit einer geringen Geschwindigkeit bis zum Erreichen einer vorbestimmten und kalibrierten Bruchkraft auf die Tablette zu und klemmt diese ein, ohne dass diese zerbricht. Dabei werden die Abmessungen der Tablette bestimmt. Nach Bestimmung der Abmessungen der Tablette wird die Bruchkraft dann langsam gesteigert, bis die Tablete zerbricht. Dabei wird dann die Bruchkraft gemessen. Für dieses Messverfahren ist eine regelmäßige und genaue Kalibrierung der Geschwindigkeit und der Bruchkraft der Bruchbacke eines Tablettenmessgerätes unerlässlich. Die Kalibrierung der Geschwindigkeit und der Bruchkraft der Bruchbacke eines Tablettenmessgerätes sind mit dem Verfahren der vorliegenden Erfindung gut möglich.

Die Erfindung besitzt den Vorteil, eine einfache und zuverlässige Vorrichtung zur Verfügung zu stellen, die transportabel ist und durch welche eine Kalibrierung der Geschwindigkeit oder der Bruchkraft einer Bruchbacke in einem Tablettenprüfgerät innerhalb kurzer Zeit möglich ist. Dadurch kann eine Kalibrierung durchgeführt werden, mit der Zeit eingespart wird und die einfach und ohne Fehlerquellen durchführbar ist.

Die Erfindung wird anhand von vier Zeichnungen beschrieben, wobei die Zeichnungen nur Ausführungsformen darstellen und die Erfindung nicht auf diese beschränkt ist.

Die Zeichnung FIG.1 zeigt eine erfindungsgemäße Vorrichtung, mit der bereits bestehende Tablettenprüfgeräte nachgerüstet werden können. Die Zeichnung FIG.2 zeigt die Prüfkammer eines Tablettenprüfgerätes, welches mit der erfindungsgemäßen Vorrichtung ausgestattet ist, in der Ansicht schräg von oben. Die Zeichnung FIG.3 zeigt die Prüfkammer eines Tablettenprüfgerätes, welches mit der erfindungsgemäßen Vorrichtung ausgestattet ist, in seitlicher Ansicht. Die Zeichnung FIG.4 zeigt eine erfindungsgemäße Vorrichtung, welche zum Aufsetzen auf ein Tablettenprüfgerät mittels Steckverbindungen vorgesehen ist.

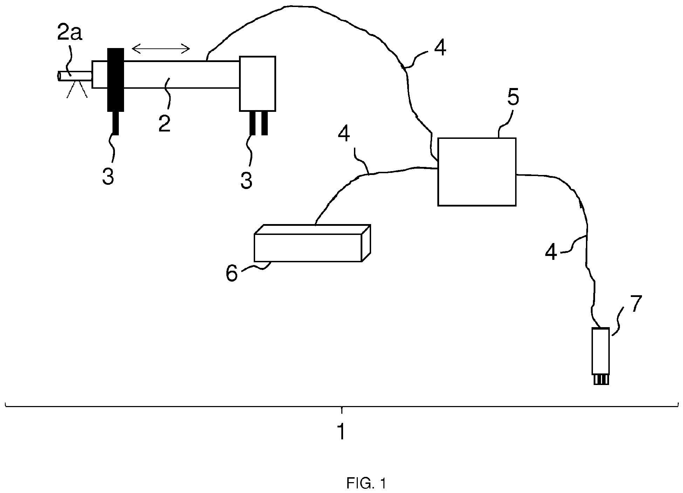

In FIG.1 ist eine erfindungsgemäße transportable Vorrichtung **(1)** zur Kalibrierung von Tablettenprüfgeräten ohne Tablettenprüfgerät gezeigt. Diese besteht aus einer Wegstreckenerfassungseinheit **(2),** welche mit Steckverbindungen **(3)** ausgestattet ist, wobei die Wegstreckenerfassungseinheit **(2)** über Kabel als Verbindungsmittel **(4)** mit einer Rechnereinheit **(5)** verbunden ist, einer Kraftmessdose **(6),** welche ebenfalls über Kabel als Verbindungsmittel **(4)** mit der Rechnereinheit **(5)** verbunden ist, und einem USB-Stecker als Übertragungsverbindung **(7),** welcher ebenfalls über Kabel **(4)** mit der Rechnereinheit **(5)** verbunden ist. Über den USB-Stecker **(7)** kann die Vorrichtung **(1)** mit dem Tablettenprüfgerät **(8)** verbunden werden. Über die Steckverbindungen **(3)** kann die Wegstreckenerfassungseinheit auf das Tablettenprüfgerät **(8)** aufgesetzt werden. Die Wegstreckenerfassungseinheit **(2)** ist hier zur Messung der Wegstrecke mit einem Lasermessgerät **(2a)** ausgestattet.

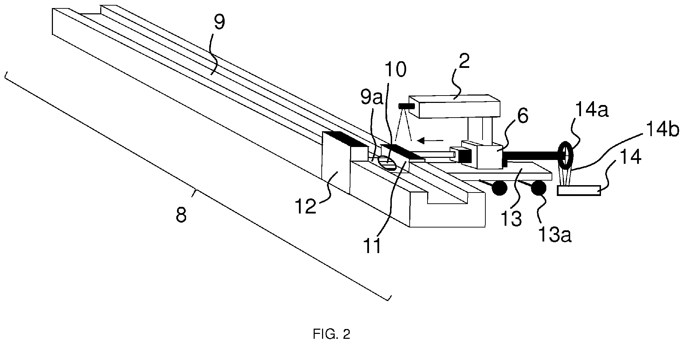

In FIG.2 ist die Prüfkammer **(9a)** eines Tablettenprüfgerätes **(8)** gezeigt, welches mit der erfindungsgemäßen Vorrichtung **(1)** kalibrierbar ist. Die Prüfkammer **(9a)** wird durch eine Positionierungsfläche **(9)** gebildet, in welche nach der Vereinzelung eine einzelne Tablette **(10)** zur Tablettenprüfung geführt wird. Die Tablette **(10)** wird durch ein Positionierungsgerät (nicht dargestellt) in der Prüfkammer **(9a)** zwischen Bruchbacke **(11)** und Gegenbacke **(12)** ausgerichtet. Danach fährt die Bruchbacke **(11)** in Pfeilrichtung auf die Gegenbacke **(12)** zu, bis die Tablette **(10)** zwischen Bruchbacke **(11)** und Gegenbacke **(12)** eingeklemmt ist. Die Wegstrecke, die die Bruchbacke **(11)** dabei zurücklegt, wird mit einem optischen Gerät als Wegstreckenerfassungseinheit **(2)** bestimmt. Durch die verbleibende Wegstrecke bis zur Gegenbacke **(12)** kann eine Abmessung, also im Fall einer länglichen Tablette **(10)** beispielsweise die Breite, bestimmt werden. Nach dem Einklemmen und der Messung einer oder mehrerer Abmessungen der Tablette **(10)** wird die Bruchkraft der Bruchbacke **(11)** solange erhöht, bis die Tablette **(10)** zerbricht. Die dabei aufgewendete Bruchkraft wird durch eine Kraftmessdose **(6)** bestimmt. Die Bruchbacke **(11)** sitzt auf einem Schlitten **(13),** der durch eine Rollvorrichtung **(13a)** in Richtung der Prüfkammer **(9a)** beweglich ist. Der Schlitten **(13)** wird durch einen Antrieb **(14)** angetrieben. Dieser sitzt hinter dem Schlitten **(13)** und treibt über eine Riemenspindel **(14a)** und einen Riemen **(14b)** den Schlitten **(13)** an, wodurch der Schlitten **(13)** mit der Bruchbacke **(11)** auf die Gegenbacke **(12)** zubewegt oder von dieser wegbewegt wird.

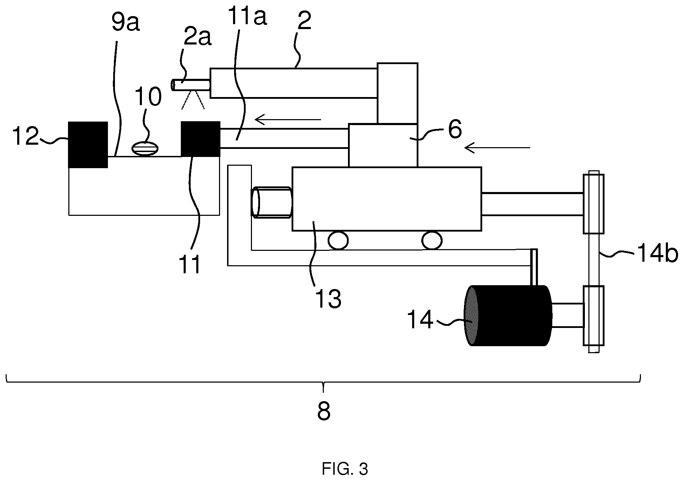

In FIG.3 ist das Tablettenprüfgerät **(8)** in seitlicher Ansicht gezeigt. Gezeigt sind zunächst die Prüfkammer **(9a),** die Bruchbacke **(11)** und die Gegenbacke **(12),** wobei die Bruchbacke **(11)** über ein Verbindungsstück (**11a**) mit einer Kraftmessdose **(6)** verbunden ist. Die Kraftmessdose **(6)** sitzt auf einem Schlitten **(13),** der über einen Riemen **(14b)** von einem Motor als Antrieb **(14)** angetrieben wird. Der Schlitten **(13)** ist in Pfeilrichtung beweglich. Zwischen der Bruchbacke **(11)** und der Gegenbacke **(12)** ist eine Tablette **(10)** positioniert worden. Bei der Bewegung des Schlittens **(13)** in Pfeilrichtung wird die Bruchbacke **(11)** in Richtung der Gegenbacke **(12)** bewegt. Die Bewegung wird solange weitergeführt, bis die Tablette **(10)** zwischen der Gegenbacke **(12)** und der Bruchbacke **(11)** eingeklemmt ist. Die Bruchkraft wird nur bis zu einem bestimmten Wert geführt, bei dem die Tablette **(10)** nicht zerbricht. Beispielhaft werden die Abmessungen der Tablette **(10)** durch die zurückgelegte Wegstrecke bestimmt. Die zurückgelegte Wegstrecke wird optisch durch ein optisches Wegstreckenerfassungseinheit **(2)** bestimmt. Dieses ist hier beispielhaft ein Lasermessgerät **(2a).** Aus der zurückgelegten Wegstrecke wird pro Zeiteinheit die Geschwindigkeit bestimmt. Diese wird gespeichert, und zur Berechnung eines optimalen Wertes für die Geschwindigkeit eingesetzt. Dieser optimale Wert wird dann bei allen folgenden Tablettenmessungen als Kalibrierungswert eingesetzt.

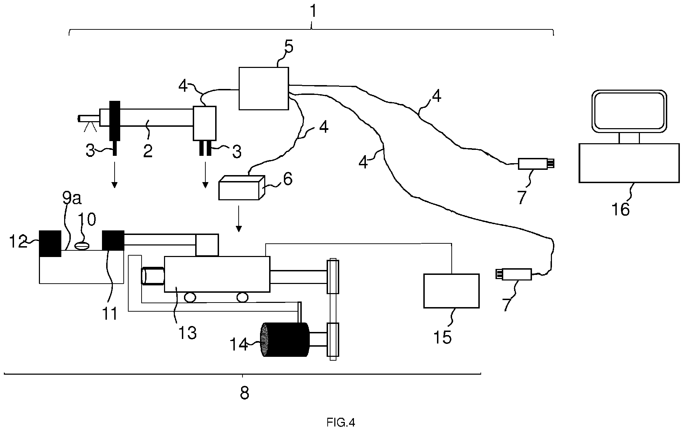

In FIG.4 ist eine erfindungsgemäße Vorrichtung **(1)** zur Kalibrierung gezeigt, welche in Pfeilrichtung auf ein Tablettenprüfgerät **(8)** aufgesteckt wird. Gezeigt sind in der Vorrichtung **(1)** die Wegstreckenerfassungeinheit **(2),** die Rechnereinheit **(5),** die Kraftmessdose **(6),** die Verbindungsmittel **(4)** und die Übertragungsverbindungen **(7).** Gezeigt sind in dem Tablettenprüfgerät **(8)** die Prüfkammer **(9a),** eine Tablette **(10)** in der Prüfkammer **(9a),** die Bruchbacke **(11),** die Gegenbacke **(12),** der Schlitten **(13),** und die Antriebseinheit für den Schlitten **(14).** Das Tablettenprüfgerät **(8)** ist ferner mit einer Rechnereinheit **(15)** zur Messung ausgestattet. Gezeigt ist weiterhin ein PC als externer Rechner **(16),** in dem Kalibrierungswerte als Referenzwerte gespeichert sind. Zur Kalibrierung wird die Vorrichtung **(1)** in Pfeilrichtung auf das Tablettenprüfgerät **(8)** aufgesteckt. Die Vorrichtung **(1)** wird dann über die Übertragungsverbindung **(7),** beispielsweise einen USB-Stecker, mit dem Rechner **(15)** des Tablettenprüfgerätes **(8)** und dem PC als externem Rechner **(16)** verbunden. Dadurch kann die Kalibrierung gegen gespeicherte Kalibrierungswerte vorgenommen werden. Nach der Kalibrierung wird die Vorrichtung **(1)** zur Kalibrierung wieder vom Tablettenprüfgerät **(8),** dem Rechner des Tablettenprüfgeräts **(15)** und vom dem PC als externem Rechner **(16)** getrennt.

**Bezugszeichenliste**

| | |
|---|---|
| 1 | Vorrichtung |
| 2 | Wegstreckenerfassungseinheit |
| 2a | Lasermessgerät |
| 3 | Steckverbindung |
| 4 | Verbindungsmittel |
| 5 | Rechnereinheit |
| 6 | Kraftmessdose |
| 7 | Übertragungsverbindung |
| 8 | Tablettenprüfgerät |
| 9 | Positionierungsfläche |
| 9a | Prüfkammer |
| 10 | Tablette |
| 11 | Bruchbacke |
| 11a | Verbindungsstück zwischen Bruchbacke und Kraftmessdose |
| 12 | Gegenbacke |
| 13 | Schlitten |
| 13a | Rollvorrichtung |
| 14 | Antrieb |
| 14a | Riemenspi ndel |
| 14b | Riemen |
| 15 | Rechner des Tablettenprüfgeräts |
| 16 | Externer Rechner |

## Patentansprüche

1. Vorrichtung (1) zur Kalibrierung eines Tablettenprüfgeräts (8), welche ein Messsystem von Geschwindigkeit oder Bruchkraft einer Bruchbacke (11) in einem Tablettenprüfgerät (8) bildet, umfassend
• mindestens ein Messgerät (2) zur Erfassung von Geschwindigkeit oder Bruchkraft einer Bruchbacke (11) des Tablettenprüfgeräts (8),
• eine Rechnereinheit (5) zur Erfassung und Auswertung der bei der Erfassung erhaltenen Daten,
**dadurch gekennzeichnet, dass**
• diese eine Übertragungsverbindung (7) zu einem Tablettenprüfgerät (8), welches eine Anzeigeeinheit und eine Eingabeeinheit enthält, umfasst, und
• Verbindungsmittel (4) zwischen dem oder den Messgeräten (2,6), der Rechnereinheit (5) und der Übertragungsverbindung (7), so dass
• die Vorrichtung (1) über die Übertragungsverbindung (7) mit dem Tablettenprüfgerät (8) verbindbar ist, und damit die über die Messgeräte (2,6) erhaltenen Daten über die Übertragungsverbindung (7) an das Tablettenprüfgerät (8) übertragbar sind und dadurch auf dessen Anzeigeeinheit sichtbar und über dessen Eingabeeinheit bearbeitbar sind, und
• die Rechnereinheit (5) zur Auswertung der bei der Messung von Geschwindigkeit oder Bruchkraft erhaltenen Daten nutzbar ist und dadurch der Antrieb (14) der Bruchbacke (11) des Tablettenprüfgeräts (8) steuerbar und kalibrierbar ist.

2. Vorrichtung (1) nach Patentanspruch 1, **dadurch gekennzeichnet, dass** es sich bei dem oder den Messgeräten (2,6) um eine Wegstreckenerfassungseinheit (2), eine Kraftmessdose (6) oder um beides handelt.

3. Vorrichtung (1) nach Patentanspruch 2, **dadurch gekennzeichnet, dass** es sich bei der Wegstreckenerfassungseinheit (2) um ein optisches Messgerät oder um ein Lasermessgerät (2a) handelt.

4. Vorrichtung (1) nach Patentanspruch 2, **dadurch gekennzeichnet, dass** es sich bei der Wegstreckenerfassungseinheit (2) um eine Reihe von elektrischen Kontakten handelt, die zur Messung der Wegstrecke der Bruchbacke (11) dienen.

5. Vorrichtung (1) nach einem der Patentansprüche 1 bis 4, **dadurch gekennzeichnet, dass** es sich bei der Übertragungsverbindung (7) zu einem Tablettenprüfgerät (8) um eine USB-Steckverbindung handelt.

6. Vorrichtung (1) nach einem der Patentansprüche 1 bis 5, **dadurch gekennzeichnet, dass** es sich bei den Verbindungsmitteln (4) zwischen dem oder den Messgeräten (2,6), der Rechnereinheit (5) und der Übertragungsverbindung (7) um elektrische Kabel handelt.

7. Vorrichtung (1) nach einem der Patentansprüche 1 bis 6, **dadurch gekennzeichnet, dass** es sich bei der Rechnereinheit (5) um eine Rechnereinheit (5) handelt, die die bei einer Messung erhaltenen Daten zu Geschwindigkeit oder Bruchkraft selbstständig auswertet und an die Anzeigeeinheit und die Eingabeeinheit des Tablettenprüfgeräts (8) überträgt.

8. Vorrichtung (1) nach einem der Patentansprüche 1 bis 6, **dadurch gekennzeichnet, dass** es sich bei der Rechnereinheit (5) um eine Verteilerdose handelt, die die bei einer Messung erhaltenen Daten zu Geschwindigkeit oder Bruchkraft an den Rechner des Tablettenprüfgeräts (8) überträgt.

9. Vorrichtung (1) nach einem der Patentansprüche 1 bis 8, **dadurch gekennzeichnet, dass** mit der Vorrichtung (1) eine Kalibrierung von Geschwindigkeit oder Bruchkraft oder beidem möglich ist.

10. Vorrichtung (1) nach einem der Patentansprüche 1 bis 8, **dadurch gekennzeichnet, dass** mit der Vorrichtung (1) eine Aufzeichnung von Geschwindigkeit oder Bruchkraft oder beidem möglich ist.

11. Vorrichtung (1) nach einem der Patentansprüche 1 bis 10, **dadurch gekennzeichnet, dass** diese zur Kalibrierung auf ein Tablettenprüfgerät (8) aufsetzbar oder aufschraubbar ist.

12. Verfahren zur Kalibrierung von Geschwindigkeit oder Bruchkraft der Bruchbacke (11) eines Tablettenprüfgeräts (8), in dem
• in einem Tablettenprüfgerät (8) eine zur Messung vorgesehene Tablette (10) in eine Prüfkammer (9a) zwischen einer Gegenbacke (12) und einer Bruchbacke (11) des Tablettenprüfgeräts (8) gegeben wird,
• die Bruchbacke (11) mit einer bestimmten Geschwindigkeit auf die Gegenbacke (12) des Tablettenprüfgeräts (8) zufährt,
**dadurch gekennzeichnet, dass**
• das Tablettenprüfgerät (8) mit einer Vorrichtung (1) zur Kalibrierung nach einem der Ansprüche 1 bis 10, welche ein mindestens ein Messgerät (2,6) zur Erfassung von Geschwindigkeit oder Bruchkraft einer Bruchbacke (11), eine Rechnereinheit (5), eine Übertragungsverbindung (7), und Verbindungsmittel (4) zwischen dem oder den Messgeräten (2,6), der Rechnereinheit (5) und der Übertragungsverbindung (7) umfasst, verbunden wird,
• bei der Messung die von der Bruchbacke (11) zurückgelegte Wegstrecke pro Zeiteinheit oder die Bruchkraft erfasst wird und dadurch die Geschwindigkeit oder die Bruchkraft der Bruchbacke (11) gemessen und gegen gespeicherte Werte kalibriert wird, und
• das Tablettenprüfgerät (8) nach der Kalibrierung wieder von der Vorrichtung (1) zur Kalibrierung getrennt wird.

13. Verfahren nach Patentanspruch 12, **dadurch gekennzeichnet, dass** durch das Verfahren die Bruchkraft und die Geschwindigkeit der Bruchbacke (11) gemessen und gegen gespeicherte Werte kalibriert werden.

14. Verfahren nach einem der Patentansprüche 12 oder 13, **dadurch gekennzeichnet, dass** die Kalibrierung gegen Kalibrierungswerte durchgeführt wird, die in der Rechnereinheit (5) gespeichert sind.

15. Verfahren nach einem der Patentansprüche 12 bis 14, **dadurch gekennzeichnet, dass** die Kalibrierungswerte, gegen die kalibriert wird, in einem externen Rechner (16) gespeichert sind und die Vorrichtung (1) mit dem externen Rechner (16) und mit dem Rechner (15) des Tablettenprüfgerätes (8) verbunden wird, um eine Kalibrierung durchzuführen.

16. Verfahren nach einem der Patentansprüche 12 bis 15, **dadurch gekennzeichnet, dass** die Geschwindigkeit der Bruchbacke (11) durch die Kalibrierung auf einen Wert von 0,1 mm/Sekunde bis 3,0 mm/Sekunde eingestellt wird.

17. Verfahren nach einem der Patentansprüche 12 bis 16, **dadurch gekennzeichnet, dass** bei der Kalibrierung eine Messung auf Linearitätsabweichungen erfolgt, indem innerhalb der Wegstrecke der Bruchbacke (11) eine Messung von bis zu drei Punkten in einer Geraden durchgeführt wird.

18. Verfahren nach einem der Patentansprüche 12 bis 17, **dadurch gekennzeichnet, dass** die Vorrichtung (1) während der Kalibrierung auf das Tablettenprüfgerät (8) aufgesetzt oder aufgeschraubt wird.

19. Verfahren zur Messung von Tabletten (10), **dadurch gekennzeichnet, dass** dabei ein mit einer Vorrichtung (1) nach einem der Ansprüche 1 bis 11 kalibriertes Tablettenmessgerät (8) zum Einsatz kommt.

20. Verfahren nach Patentanspruch 19, **dadurch gekennzeichnet, dass** nach der Kalibrierung der Geschwindigkeit und der Bruchkraft der Bruchbacke (11) auch die Abmessungen der Tablette (10) bestimmt werden.

## Geänderte Patentansprüche

### Geänderte Patentansprüche gemäss Regel 137(2) EPÜ.

1. Vorrichtung (1) zur Kalibrierung eines Tablettenprüfgeräts (8), welche ein Messsystem von Geschwindigkeit oder Bruchkraft einer Bruchbacke (11) in einem Tablettenprüfgerät (8) bildet, umfassend
• mindestens ein Messgerät (2) zur Erfassung von Geschwindigkeit oder Bruchkraft einer Bruchbacke (11) des Tablettenprüfgeräts (8),
• eine Rechnereinheit (5) zur Erfassung und Auswertung der bei der Erfassung erhaltenen Daten,
**dadurch gekennzeichnet, dass**
• diese eine Übertragungsverbindung (7) zu einem Tablettenprüfgerät (8), welches eine Anzeigeeinheit und eine Eingabeeinheit enthält, umfasst, und
• Verbindungsmittel (4) zwischen dem oder den Messgeräten (2,6), der Rechnereinheit (5) und der Übertragungsverbindung (7), so dass
• die Vorrichtung (1) über die Übertragungsverbindung (7) mit dem Tablettenprüfgerät (8) verbindbar ist, und damit die über die Messgeräte (2,6) erhaltenen Daten über die Übertragungsverbindung (7) an das Tablettenprüfgerät (8) übertragbar sind und dadurch auf dessen Anzeigeeinheit sichtbar und über dessen Eingabeeinheit bearbeitbar sind, und
• die Rechnereinheit (5) aus einem Rechner besteht, der die zur Kalibierung erforderlichen Daten enthält, gegen die die Kalibrierung dann erfolgt und so die Rechnereinheit (5) zur Auswertung der bei der Messung von Geschwindigkeit oder Bruchkraft erhaltenen Daten nutzbar ist und dadurch der Antrieb (14) der Bruchbacke (11) des Tablettenprüfgeräts (8) steuerbar und kalibrierbar ist.

2. Vorrichtung (1) nach Patentanspruch 1, **dadurch gekennzeichnet, dass** es sich bei dem oder den Messgeräten (2,6) um eine Wegstreckenerfassungseinheit (2), eine Kraftmessdose (6) oder um beides handelt.

3. Vorrichtung (1) nach Patentanspruch 2, **dadurch gekennzeichnet, dass** es sich bei der Wegstreckenerfassungseinheit (2) um ein optisches Messgerät oder um ein Lasermessgerät (2a) handelt.

4. Vorrichtung (1) nach Patentanspruch 2, **dadurch gekennzeichnet, dass** es sich bei der Wegstreckenerfassungseinheit (2) um eine Reihe von elektrischen Kontakten handelt, die zur Messung der Wegstrecke der Bruchbacke (11) dienen.

5. Vorrichtung (1) nach einem der Patentansprüche 1 bis 4, **dadurch gekennzeichnet, dass** es sich bei der Übertragungsverbindung (7) zu einem Tablettenprüfgerät (8) um eine USB-Steckverbindung handelt.

6. Vorrichtung (1) nach einem der Patentansprüche 1 bis 5, **dadurch gekennzeichnet, dass** es sich bei den Verbindungsmitteln (4) zwischen dem oder den Messgeräten (2,6), der Rechnereinheit (5) und der Übertragungsverbindung (7) um elektrische Kabel handelt.

7. Vorrichtung (1) nach einem der Patentansprüche 1 bis 6, **dadurch gekennzeichnet, dass** es sich bei der Rechnereinheit (5) um eine Rechnereinheit (5) handelt, die die bei einer Messung erhaltenen Daten zu Geschwindigkeit oder Bruchkraft selbstständig auswertet und an die Anzeigeeinheit und die Eingabeeinheit des Tablettenprüfgeräts (8) überträgt.

8. Vorrichtung (1) nach einem der Patentansprüche 1 bis 6, **dadurch gekennzeichnet, dass** es sich bei der Rechnereinheit (5) um eine Verteilerdose handelt, die die bei einer Messung erhaltenen Daten zu Geschwindigkeit oder Bruchkraft an den Rechner des Tablettenprüfgeräts (8) überträgt.

9. Vorrichtung (1) nach einem der Patentansprüche 1 bis 8, **dadurch gekennzeichnet, dass** mit der Vorrichtung (1) eine Kalibrierung von Geschwindigkeit oder Bruchkraft oder beidem möglich ist.

10. Vorrichtung (1) nach einem der Patentansprüche 1 bis 8, **dadurch gekennzeichnet, dass** mit der Vorrichtung (1) eine Aufzeichnung von Geschwindigkeit oder Bruchkraft oder beidem möglich ist.

11. Vorrichtung (1) nach einem der Patentansprüche 1 bis 10, **dadurch gekennzeichnet, dass** diese zur Kalibrierung auf ein Tablettenprüfgerät (8) aufsetzbar oder aufschraubbar ist.

12. Verfahren zur Kalibrierung von Geschwindigkeit oder Bruchkraft der Bruchbacke (11) eines Tablettenprüfgeräts (8), in dem
• in einem Tablettenprüfgerät (8) eine zur Messung vorgesehene Tablette (10) in eine Prüfkammer (9a) zwischen einer Gegenbacke (12) und einer Bruchbacke (11) des Tablettenprüfgeräts (8) gegeben wird,
• die Bruchbacke (11) mit einer bestimmten Geschwindigkeit auf die Gegenbacke (12) des Tablettenprüfgeräts (8) zufährt,
**dadurch gekennzeichnet, dass**
• das Tablettenprüfgerät (8) mit einer Vorrichtung (1) zur Kalibrierung nach einem der Ansprüche 1 bis 11, welche ein mindestens ein Messgerät (2,6) zur Erfassung von Geschwindigkeit oder Bruchkraft einer Bruchbacke (11), eine Rechnereinheit (5), eine Übertragungsverbindung (7), und Verbindungsmittel (4) zwischen dem oder den Messgeräten (2,6), der Rechnereinheit (5) und der Übertragungsverbindung (7) umfasst, verbunden wird,
• bei der Messung die von der Bruchbacke (11) zurückgelegte Wegstrecke pro Zeiteinheit oder die Bruchkraft erfasst wird und dadurch die Geschwindigkeit oder die Bruchkraft der Bruchbacke (11) gemessen und gegen gespeicherte Werte kalibriert wird, und
• das Tablettenprüfgerät (8) nach der Kalibrierung wieder von der Vorrichtung (1) zur Kalibrierung getrennt wird, und
• die Kalibrierung gegen Kalibrierungswerte durchgeführt wird, die in der Rechnereinheit (5) gespeichert sind.

13. Verfahren nach Patentanspruch 12, **dadurch gekennzeichnet, dass** durch das Verfahren die Bruchkraft und die Geschwindigkeit der Bruchbacke (11) gemessen und gegen gespeicherte Werte kalibriert werden.

14. Verfahren nach einem der Patentansprüche 12 oder 13, **dadurch gekennzeichnet, dass** die Kalibrierungswerte, gegen die kalibriert wird, in einem externen Rechner (16) gespeichert sind und die Vorrichtung (1) mit dem externen Rechner (16) und mit dem Rechner (15) des Tablettenprüfgerätes (8) verbunden wird, um eine Kalibrierung durchzuführen.

15. Verfahren nach einem der Patentansprüche 12 bis 14, **dadurch gekennzeichnet, dass** die Geschwindigkeit der Bruchbacke (11) durch die Kalibrierung auf einen Wert von 0,1 mm/Sekunde bis 3,0 mm/Sekunde eingestellt wird.

16. Verfahren nach einem der Patentansprüche 12 bis 15, **dadurch gekennzeichnet, dass** bei der Kalibrierung eine Messung auf Linearitätsabweichungen erfolgt, indem innerhalb der Wegstrecke der Bruchbacke (11) eine Messung von bis zu drei Punkten in einer Geraden durchgeführt wird.

17. Verfahren nach einem der Patentansprüche 12 bis 16, **dadurch gekennzeichnet, dass** die Vorrichtung (1) während der Kalibrierung auf das Tablettenprüfgerät (8) aufgesetzt oder aufgeschraubt wird.

18. Verfahren zur Messung von Tabletten (10), **dadurch gekennzeichnet, dass** dabei ein mit einer Vorrichtung (1) nach einem der Ansprüche 1 bis 11 kalibriertes Tablettenmessgerät (8) zum Einsatz kommt.

19. Verfahren nach Patentanspruch 18, **dadurch gekennzeichnet, dass** nach der Kalibrierung der Geschwindigkeit und der Bruchkraft der Bruchbacke (11) auch die Abmessungen der Tablette (10) bestimmt werden.
